# EUROPÄISCHE PATENTANMELDUNG

(11) **EP 1 970 708 A1**
(43) Veröffentlichungstag der Anmeldung: **17.09.2008**
(21) Anmeldenummer: 07004665.1
(22) Anmeldetag: 07.03.2007
(51) Int. Cl.: G01N 33/569

(54) **Biochip und Verfahren zum selektiven Nachweis von Chlamydia trachomatis-Infektionen**

(71) Anmelder: Mikrogen Molekularbiologische Entwicklungs-GmbH, 82061 Neuried (DE)
(72) Erfinder: Forsbach-Birk, Vera, 89075 Ulm (DE); Simnacher, Ulrike, 89075 Ulm (DE); Essig, Andreas, 89079 Ulm (DE); Pfrepper, Klaus-Ingmar, 81375 München (DE)
(74) Vertreter: Keller, Günter

(57) **Zusammenfassung**

Die vorliegende Erfindung betrifft ein Verfahren zum selektiven Nachweis von *Chlamydia* trachomatis-Infektionen, worin die Antigene CT017, CT098, CT318-L1P, CT431, CT456-TARP, CT603-TSAP und CT664 zum spezifischen Nachweis von *Chlamydia trachomatis-*Antikörpern in Proben von Säugetieren verwendet werden. Das erfindungsgemäße Verfahren ermöglicht ein selektives Nachweisverfahren von *Chlamydia trachomatis-*Infektionen, wobei keine falsch-positiven Ergebnisse durch andere *Chlamydia*-Spezies wie beispielsweise *Chlamydia pneumoniae* erzeugt werden.

Des Weiteren betrifft die Erfindung einen Biochip, der die vorstehend genannten *Chlamydia trachomatis*-spezifischen Antigene zum Nachweis von Antikörpern aufweist. Insbesondere ist der Biochip mit den erfindungsgemäßen Antigenen für Multiparameter-Nachweisverfahren geeignet.

## Beschreibung

Die vorliegende Erfindung betrifft ein Verfahren zum selektiven Nachweis von *Chlamydia trachomatis*-Infektionen, worin die Antigene CT017, CT098, CT318-L1P, CT431, CT456-TARP, CT603-TSAP und CT664 zum Nachweis von *Chlamydia trachomatis*-spezifischen Antikörpern in Proben von Säugetieren verwendet werden. Des Weiteren betrifft die Erfindung einen Biochip, der die vorstehend genannten Antigene zum Nachweis der *Chlamydia trachomatis*-spezifischen Antikörper aufweist. Die vorliegende Erfindung betrifft zudem ein Test-Kit, das zum Nachweis von *Chlamydia trachomatis*-Infektionen geeignet ist.

Chlamydien sind weltweit verbreitete Infektionserreger von erheblicher klinischer und epidemiologischer Relevanz. Es handelt sich um obligat intrazelluläre replizierende Bakterien, die morphologisch und funktionell in zwei unterschiedlichen Zellformen vorliegen (hochinfektiöse, stoffwechselinaktive Elementarkörper und replikationsfähige, intrazelluläre Retikularkörper) und einen typischen Entwicklungszyklus durchlaufen. Die Zielzellen der Erreger sind in der Regel die Epithelzellen der Konjunktiva sowie der Schleimhäute des Respirationstraktes und des Genitaltraktes.

Das Erregerreservoir für *Chlamydia trachomatis* und *Chlamydia pneumoniae* (neue Nomenklatur auch: *Chlamydophila pneumoniae*) ist der Mensch, während für *Chlamydia psittaci* (neue Nomenklatur auch: *Chlamydophila psittaci*) vor allem Vögel aber auch Nutztiere wichtige Infektionsquellen sein können. Aufgrund ihrer intrazellulären Lage haben Chlamydien Strategien entwickelt, sich der Wirtsabwehr zu entziehen, so dass eine Chronifizierung der Infektion mit der Gefahr von Folgeerkrankungen nicht selten ist. In solchen Fällen ist die Diagnose durch den Erregernachweis meist nicht mehr möglich, wohingegen der Nachweis speziesspezifischer Antikörper erhebliche diagnostische Bedeutung erlangt.

Chlamydien können sich auch in Zellen der Wirtsabwehr wie Monozyten und Lymphozyten vermehren. Möglicherweise gelingt es den Erregern dadurch vom Ort der Primärinfektion zu disseminieren und somit in andere Kompartimente (Gelenke, Gefäße) zu gelangen. Die Primärinfektion verläuft klinisch oft uncharakteristisch und oligosymptomatisch, wodurch die Ausbreitung und die Weiterverbreitung der Infektion begünstigt wird. Da eine Primärinfektion offenbar keine vollständige Immunität induziert, sind Reinfektionen gerade bei *Chlamydia pneumoniae* und *Chlamydia trachomatis* nicht selten. Dementsprechend ist die Therapie oft langwierig und gelingt nur mit Antibiotika, die intrazellulär hohe Wirkstoffspiegel erreichen. Gerade wegen ihrer uncharakteristischen klinischen Symptomatik spielt für den Nachweis von Chlamydien-Infektionen die Labordiagnostik eine entscheidende Rolle.

*Chlamydia trachomatis* gehört in den Industrienationen zu den häufigsten Ursachen von sexuell übertragbaren Erkrankungen (STD). Nach Schätzungen des US-amerikanischen "Center of Disease Control and Prevention" sind mehr als 700 Millionen Menschen mit *Chlamydia trachomatis* infiziert. Die jährliche Inzidenz liegt bei 50 Millionen. Deutschlandweit wird mit einer jährlichen Inzidenz von 300.000 gerechnet (insgesamt ca. 1,15 Millionen). In den Entwicklungsländern verursacht der Erreger mit dem Trachom eine chronische Keratokonjunktivitis, die nach wie vor in diesen Ländern die häufigste Ursache der vermeidbaren Erblindung ist.

Das Erkrankungsspektrum von *Chlamydia trachomatis* ist abhängig vom Serotyp des Erregers. Die Serotypen A-C verursachen das Trachom, die Serotypen L1-L3 verursachen das Lymphogranuloma venereum, eine invasiv in Stadien verlaufende sexuell übertragbare Erkrankung mit vorwiegendem Vorkommen in den Tropen. Die Serotypen D-K dagegen spielen in den Industrienationen die entscheidende Rolle als Ursache von urogenitalen Infektionen, welche zur infektionsbedingten Sterilität und postinfektiösen Arthritis führen können. Die perinatale Übertragung des Erregers von der infizierten Mutter auf das Neugeborene führt zur sogenannten Neugeborenkonjunktivitis oder auch zur Neugeborenenpneumonie. Aufgrund der schweren Folgeerkrankungen, die unerkannte und damit unbehandelte *Chlamydia trachomatis-*Infektionen verursachen können, werden in skandinavischen und angloamerikanischen Ländern Screeninguntersuchungen für Risikogruppen angeboten. Ob in Deutschland auch Screeninguntersuchungen eingeführt werden, ist derzeit noch unklar.

*Chlamydia pneumoniae* ist ein erst seit ca. 15 Jahren bekannter Erreger, der typischerweise Infektionen des Respirationstraktes wie Sinusitis, Pharyngitis, Bronchitis und Pneumoniae hervorrufen kann. Seroepidemiologische Studien weisen darauf hin, dass *Chlamydia pneumoniae* bei Antikörperprävalenzen von über 50% weit verbreitet ist. Letztlich ist jedoch die klinische Relevanz von *Chlamydia pneumoniae* nicht abschließend geklärt. Im Rahmen des BMBF-Kompetenznetzwerkes "Ambulant erworbene Pneumonie" (CAPNETZ) wird derzeit versucht die Bedeutung des Erregers bei der ambulant erworbenen Pneumoniae zu klären, was sich allerdings durch methodische Schwierigkeiten in der Labordiagnostik insbesondere in der Serologie als extrem schwierig herausstellt. Noch schwieriger jedoch ist es, die Relevanz des Erregers bei extrapulmonalen Erkrankungen abzuschätzen. Von zentraler nicht zuletzt auch gesundheitspolitischer und volkswirtschaftlicher Bedeutung ist die Frage, ob *Chlamydia pneumoniae* an der Atherogenese beteiligt und damit mitverantwortlich ist für die Volkskrankheiten Herzinfarkt und Schlaganfall.

*Chlamydia psittaci* ist der Erreger der Ornithose oder auch der Papageienkrankheit. Es handelt sich dabei um eine Zooanthroponose, die in der Regel von infizierten Zier- oder Nutzvögeln durch Ausscheidung erregerhaltiger Sekrete und Exkremente, aerogen oder seltener durch direkten Kontakt, auf den Menschen übertragen wird. Infektionsgefährdet sind neben den Haltern von Zier- und Nutzvögeln daher vor allem Tierhändler und in der geflügelverarbeitenden Industrie Beschäftigte. Insgesamt ist die Erkrankung in Deutschland selten geworden, allerdings ist aufgrund der schwierigen Diagnostik von einer relativ hohen Dunkelziffer auszugehen.

Hauptmanifestationsform der Infektion ist eine atypische Pneumonie, die mit einer schweren systemischen Symptomatik einhergehen kann. Unbehandelt kann die Infektion tödlich verlaufen. Eine Übertragung von Mensch zu Mensch wurde bisher nicht beobachtet, der Erreger gilt jedoch als hoch kontagiös und wird nach Biostoffverordnung in die Laborsicherheitsstufe 3 eingestuft.

Daneben hat *Chlamydia psittaci* eine große ökonomische Bedeutung im Bereich der Nutztierhaltung, wo schwere systemisch verlaufende Infektionen auftreten können und sich bei infizierten Beständen insbesondere bei Schafen aber auch anderen Nutztieren hohe Abortraten finden.

Die Diagnostik von Chlamydien-Infektionen ist generell schwierig, aufwändig und teuer. Für den Nachweis von chronischen *Chlamydia trachomatis*-Infektionen stehen bisher keine ausreichend empfindlichen, standardisierten und gut evaluierbaren Nachweisverfahren zur Verfügung, die zudem keine falsch-positiven Ergebnisse erzeugen.

Der Nachweis einer *Chlamydia trachomatis-*Infektion wurde bislang beispielsweise mit Nukleinsäure-Amplifikationstests (NAAT, nucleic acid amplification test) durchgeführt. Diese Verfahren wurden zum Nachweis von genitalen Infektionen routinemäßig in klinischen Labors durchgeführt. Derartige, kommerziell erhältliche Assays sind jedoch sehr teuer und zu komplex, um sie im großen Maßstab einzusetzen. Gerade aufgrund der hohen Selektivität dieses Testverfahrens besteht die Gefahr von Kreuzkontaminierungen bzw. falschen Testergebnissen aufgrund falscher Handhabung der Proben. Daher erfordert dieses Testverfahren eine große Aufmerksamkeit bezüglich der Qualitätskontrolle und der Schulung des Laborpersonals.

Des Weiteren besteht die Gefahr, dass sich der Erreger bzw. dessen Nukleinsäuresequenz ändert und ein Nachweis mit den bisherigen Sonden nicht mehr möglich ist. Zudem besteht die Gefahr, dass falsch-positive Ergebnisse erzeugt werden, wenn mehr als eine Chlamydia-Spezies die Nukleinsäuresequenz der Sonde aufweist.

Ein anderes bekanntes Verfahren beruht auf dem Nachweis von Serumantikörpern gegen *Chlamydia trachomatis.* Die bisher auf dem Markt befindlichen gattungsspezifischen Antikörpernachweise erfassen Antikörper gegen alle Chlamydienarten und sind angesichts der hohen Prävalenz von *Chlamydia pneumoniae*-Antikörper positiven Patienten oft sehr schwer zu interpretieren. Speziesspezifische Tests sind technisch sehr aufwändig und schwierig zu beurteilen (Mikroimmunfluoreszenztest).

In der Vergangenheit gab es bereits viele erfolglose Versuche immundominante und spezifische Chlamydien-Antigene zu identifizieren. Insbesondere hat sich die spezies-spezifische Diagnostik mittels *Chlamydia pneumoniae*-Antigenen als schwierig erwiesen. Hierbei ergaben sich Diskrepanzen in der Reaktivität zwischen dem bisher geltenden Goldstandard MIF (Mikroimmunfluoreszenztest) und den rekombinanten Antigenen (Maile et al., 2005, *"recom*Line Chlamydia: a new serological test system for the detection of antibodies against *Chlamydia trachomatis, Chlamydia pneumoniae and Chlamydia psittaci",* 3. Deutscher Chlamydienworkshop, 09.03.-11.03.2005, Jena).

Anfängliche Versuche im Rahmen der vorliegenden Erfindung zur Klonierung und rekombinanten Herstellung von bekannten Chlamydien-Proteinen, wie beispielsweise den Proteinen PGP3 (Maile et al., 2004, "Evaluation of selected recombinant chlamydial antigens for serological diagnosis of *Chlamydia trachomatis and Chlamydia pneumoniae* infection", 5th Meeting of the European Society for Chlamydia Research, 01-04.09.2004, Budapest), MOMP, OMP2, hsp60, MIP und MOMPIV von *Chlamydia trachomatis* waren für die Entwicklung eines spezifischen Nachweisverfahrens für *Chlamydia trachomatis*-Infektionen nicht geeignet.

Dies zeigt, dass mit Ganzzell-Lysat-Antigenen auf der Basis von gereinigten Chlamydien-Elementarkörperchen keine wesentlichen Fortschritte in der Chlamydien-Serodiagnostik zu erreichen sind. So sind bisher auch keine spezifischen Antigene von *Chlamydia pneumoniae* bekannt, die eine sichere serologische Differenzierung von *Chlamydia pneumoniae* und *Chlamydia trachomatis*-Infektionen zulassen.

Darüberhinaus wurde eine große Anzahl PCR-basierter Verfahren entwickelt, wobei beispielsweise unterschiedliche Zielgene von *Chlamydia pneumoniae* aus respiratorischen und nicht-respiratorischen Proben entwickelt wurden. Viele dieser PCR-Verfahren waren jedoch nicht verlässlich oder stabil genug, um reproduzierbare Ergebnisse in klinischen Routineuntersuchungen zu ermöglichen. Daher wurden diese Assays eher als Forschungswerkzeuge verwendet.

Des Weiteren wurden Enzymimmunoassay-basierende Verfahren zur Detektion von *Chlamydia trachomatis*-Infektionen mittels monoklonaler oder polyklonaler Antikörper verwendet. Diese Tests haben sich bislang jedoch nicht als erfolgreich dargestellt, insbesondere aufgrund ihrer geringen Sensitivität und der von ihnen erzeugten falsch-positiven Testergebnisse.

Eine Aufgabe der vorliegenden Erfindung besteht daher darin, ein selektives Nachweisverfahren für *Chlamydia trachomatis*-Infektionen zur Verfügung zu stellen. Dieses Verfahren soll keine Kreuzreaktivität bezüglich anderer Erreger aufweisen und dadurch insbesondere eine Abgrenzung gegenüber *Chlamydia pneumoniae*-Infektionen ermöglichen.

Insbesondere muss das Testverfahren eine hohe Sensitivität aufweisen, um den Nachweis chronischer *Chlamydia trachomatis*-Infektionen zu ermöglichen. Des Weiteren soll das Testverfahren zum selektiven Nachweis von *Chlamydia trachomatis*-Infektionen für routinemäßige Laboruntersuchungen aufgrund kostengünstiger, einfacher und sicherer Handhabung geeignet und zusätzlich nicht nur zum Nachweis des Krankheitserregers, sondern auch zur Beurteilung des Fortschritts der Krankheit bzw. des Krankheitsstadiums geeignet sein.

Das selektive Testverfahren soll insbesondere für die Anwendung in Multiparameter-Tests auf der Basis von Biochip- oder Luminex-Technologie geeignet sein, insbesondere, da Chlamydien-Infektionen auch Auslöser von Krankheitsbildern mit typischen Folgekomplikationen wie der reaktiven Arthritis sein können. Gerade die Arthritisdiagnostik ist gegenwärtig durch die Notwendigkeit unter vielen potentiellen Erregern die genaue Ursache zu finden, mit einem sehr kostenintensiven Aufwand von Einzeltests verbunden.

Eine Aufgabe ist es auch, ein Testverfahren für den veterinärmedizinischen Bereich zur Verfügung zu stellen, da auch hier ein großer Bedarf nach selektiven Tests besteht.

Die erfindungsgemäße Aufgabe wurde durch den Einsatz von individuell immobilisierten rekombinanten Antigenen anstelle von herkömmlich verwendeten Lysaten und Elementarkörperchen gelöst und stellt eine wesentliche Verbesserung im Bereich der Chlamydien-Serodiagnostik dar. Die Identifizierung der für *Chlamydia trachomatis* spezifischen Antigene ist in den Beispielen dargestellt.

Die erfolgreiche Charakterisierung immundominanter und spezies-spezifischer Chlamydien-Proteine, die als Antigene für die Diagnostik eingesetzt werden können, konnte unerwarteterweise durch ausschließliche in-vivo-Expression erreicht werden, wobei Antigene identifiziert wurden, die konventionellen diagnostischen Ansätzen entgehen. Hierbei wurden alle Antigene mittels 2D-Immunelektrophorese und Western-Blots mit Hilfe charakterisierter Patienten-Seren sowie konsekutiver massenspektrometrischer Analyse reaktiver Spots identifiziert. Dies ist ebenfalls in den Beispielen beschrieben.

Die vorteilhaft hohe Seroreaktivität der erfindungsgemäßen Antigene wurde dadurch erreicht, dass die Kandidaten-Antigene in bakteriellen Expressionsvektoren kloniert und rekombinant exprimiert wurden und nach einer eventuellen Expressionsoptimierung und Proteinreinigung in Line-Assays analysiert wurden, um eine optimale Kombination an rekombinanten Antigenen zu finden. Bei den "Line-Assays" werden mehrere meist rekombinant hergestellte Antigene als schmale Banden auf einen geeigneten Träger (bspw. Nitrocellulose) aufgebracht und die Teststreifen mit der Untersuchungsflüssigkeit (bspw. Serum) umgesetzt. Diese Antigene ermöglichen auf vorteilhafte Weise einerseits eine genaue Speziesdifferenzierung und andererseits eine Unterscheidung zwischen aktiven und abgelaufenen Chlamydien-Infektionen. Dies war bislang nur mit PCR-Verfahren möglich.
Der stadienabhängigen, spezies-spezifischen Serologie zur Diagnose von *Chlamydia trachomatis*-Infektionen kommt eine wichtige Rolle bei therapeutischen Entscheidungen aber auch bei der Bewertung der Prognose bestehender Krankheitszustände zu.

Wie bereits erwähnt, ermöglicht die Verwendung der erfindungsgemäßen Antigene in einem Multiparameter-Verfahren auf vorteilhafte Weise den gleichzeitigen Nachweis verschiedener Erreger, insbesondere den Nachweis der verschiedenen Chlamydia-Erreger, *Chlamydia trachomatis, Chlamydia pneumoniae* und *Chlamydia psittaci.* Ein Vorteil der Verwendung von mehr als einem Antigen im Nachweisverfahren liegt darin, dass die Testsicherheit erhöht wird. Die erfindungsgemäßen Antigene besitzen einerseits eine höhere Spezifität bei klinisch relevanten Infektionen mit *Chlamydia trachomatis,* sowie andererseits eine geringere Kreuzreaktivität mit *Chlamydia pneumoniae* als bislang bekannte rekombinant hergestellte Antigene (siehe Tabelle 3). Die erfindungsgemäßen Antigene waren bislang nicht im Zusammenhang mit dem selektiven Nachweis von *Chlamydia trachomatis* bekannt.

Das Antigen CT456-TARP wurde zwar bereits beschrieben (Clifton et al., Proc Natl Acad Sci USA. 2004, Jul 6;101(27):10166-71; Clifton et al., Infect Immun. 2005, Jul;73(7):3860-8; Jewett et al., Proc Natl Acad Sci U S A. 2006 Oct 17;103(42):15599-604.), jedoch nicht im Zusammenhang mit dem Nachweis von *Chlamydia trachomatis.*

Einige weitere immunreaktive Antigene von *Chlamydia trachomatis* wurden bereits beschrieben: CT858-CPAF (Sharma et al., Infect Immun. 72(12):7164-71); CT089 und CT795 (Sharma et al., Infect Immun. 74(3):1490-99) und CT813 (Chen et al., Infect Immun. 74(8):4826-40). Diese vier Proteine wurden zu Vergleichszwecken im Rahmen der vorliegenden Anmeldung untersucht (Tabelle 3). Die Ergebnisse zeigen, dass die vier bekannten Antigene CT858-CPAF, CT089, CT795 und CT813 bei Infektionen mit *Chlamydia pneumoniae* sowie bei gesunden Blutspendern mit Antikörpern gegen *Chlamydia trachomatis* häufiger als die erfindungsgemäßen Antigene reagieren (siehe Tabelle 3).

Die vorliegende Erfindung betrifft daher ein Verfahren zum selektiven Nachweis von *Chlamydia trachomatis -* Infektionen, wobei die Antigene ausgewählt werden aus der Gruppe bestehend aus: CT017, CT098, CT318-L1P, CT431, CT456-TARP, CT603-TSAPund CT664, sowie zusätzlich bevorzugt auch aus Fragmenten und Teilsequenzen der vorstehenden Antigene oder aus im Wesentlichen dazu gleiche Antigenen, die dazu verwendet werden, Antikörper in Proben zu detektieren. Bevorzugt werden mindestens ein, weiter bevorzugt mindestens zwei, noch weiter bevorzugt mindestens drei, weiter bevorzugt mindestens vier, weiter bevorzugt mindestens fünf und am meisten bevorzugt mindestens sechs Antigene aus den vorstehend genannten Gruppen von Antigenen ausgewählt. Weiter bevorzugt sind die Antigene ausgewählt aus der Gruppe bestehend aus: CT017, CT098, CT318-L1P, CT431, CT603-TSAP und CT664.

In einer bevorzugten Ausführungsform der Erfindung zum Nachweis von chronischen Erkrankungen sind die Antigene besonders bevorzugt ausgewählt aus der Gruppe bestehend aus: CT017, CT098, CT318-L1 P, CT431, CT603-TSAP und CT664. Noch weiter bevorzugt für den Nachweis von chronischen Erkrankungen bzw. Infektionen sind die Antigene aus der Gruppe ausgewählt bestehend aus: CT017, CT098, CT318-L1 P und CT664. Am meisten bevorzugt sind die Antigene: CT603-TSAP und CT664.

Eine weitere Ausführungsform der Erfindung betrifft ein Verfahren, das dadurch gekennzeichnet ist, dass die Detektion der Antikörper den Nachweis eines Antigen-/Antikörperkomplexes umfasst.

Eine bevorzugte Ausführungsform der Erfindung betrifft ein in vitro Verfahren zur Analyse von Proben von menschlichen Patienten.

In einer bevorzugten Ausführungsform des Verfahrens werden die zu untersuchenden Proben einer Zelllyse unterzogen, bevor sie mit den Antigenen in Kontakt gebracht werden. Hierdurch werden Erreger, beziehungsweise erregerspezifische Antigene aus den infizierten Zellen freigesetzt.

Bei einer weiteren bevorzugten Ausführungsform des Verfahrens erfolgt die Detektion der Antikörper mittels ELISA (Enzyme-Linked Immunosorbent Assay), Western-Blot oder Line-Assay, besonders bevorzugt ELISA. Besonders bevorzugt ist hierbei die Verwendung von ELISA, Western-Blot und Line-Assay mit peroxidasekonjugierten Sekundärantikörpern, wobei der Nachweis der Antigen-Antikörper-Reaktion über ein Färbesubstrat (TMB, Tetramethylbenzidin) erfolgt.

Eine weiter bevorzugte Ausführungsform des Verfahrens ist dadurch gekennzeichnet, dass bei dem Verfahren zum selektiven Nachweis von *Chlamydia trachomatis* Infektionen die Menge an Antikörpern in der Probe quantitativ bestimmt wird.

In einer bevorzugten Ausführungsform des Verfahrens erfolgt der Nachweis der Antigen-Antikörper-Reaktion unter Verwendung von peroxidasekonjugierten Sekundärantikörpern und dem Färbesubstrat (TMB).

Eine weitere Ausführungsform der Erfindung betrifft ein Verfahren zum Analysieren des Krankheitsverlaufs einer *Chlamydia trachomatis -* Infektion, worin in bestimmten Zeitabständen Proben von einem Patienten genommen werden und die Proben nach dem erfindungsgemäßen Verfahren untersucht werden. Bevorzugt ist hierbei, dass bei dem erfindungsgemäßen Verfahren zum selektiven Nachweis von *Chlamydia trachomatis -* Infektionen die Menge an Antikörpern in der Probe quantitativ bestimmt wird.
Des Weiteren betrifft die Erfindung ein Test-Kit zum selektiven Nachweis von *Chlamydia trachomatis -* Infektionen, dadurch gekennzeichnet, dass Antigene, ausgewählt aus der Gruppe bestehend aus: CT017, CT098, CT318-L1P, CT431, CT456-TARP, CT603-TSAP, und CT664, verwendet werden, um Antikörper in Proben von Patienten zu detektieren. Bevorzugt werden mindestens ein, weiter bevorzugt mindestens zwei, noch weiter bevorzugt mindestens vier, und am meisten bevorzugt mindestens sechs Antigene aus den vorstehend genannten Gruppen von Antigenen ausgewählt. In einer besonders bevorzugten Ausführungsform wird die gesamte Gruppe der *Chlamydia trachomatis -* spezifischen Antigenen für das Nachweisverfahren verwendet.

Zudem betrifft die Erfindung einen Biochip der Antigene, ausgewählt aus der Gruppe bestehend aus: CT017, CT098, CT318-L1P, CT431, CT456-TARP, CT603-TSAP, und CT664, für den Nachweis von *Chlamydia trachomatis* -spezifischen Antikörpern enthält. Bevorzugt werden mindestens ein, weiter bevorzugt mindestens zwei, noch weiter bevorzugt mindestens vier, und am meisten bevorzugt mindestens sechs Antigene aus den vorstehend genannten Gruppen von Antigenen ausgewählt.

Eine Ausführungsform des Biochips zeichnet sich dadurch aus, dass die Antigene, ausgewählt aus der Gruppe bestehend aus: CT017, CT098, CT318-L1P, CT431, CT456-TARP, CT603-TSAP, und CT664, auf einer Stelle eines festen Trägers, beispielsweise aus Glas oder Plastik bzw. einer Membran, bevorzugterweise einer Membran, angebracht sind und Stellen auf dem festen Träger mit andere Antigenen räumlich getrennt davon sind. Weiter bevorzugt ist hierbei, dass die Antigen-/ Antikörperkomplexe durch Farbreaktionen auf dem festen Träger, bevorzugt einer Membran, detektiert bzw. sichtbar gemacht werden können. Bevorzugterweise erfolgt der Nachweis unter Verwendung von Farbreaktionen, bevorzugterweise mit TMB (Tetramethylbenzidin), oder mittels Fluoreszenzfarbstoffen wie bevorzugterweise, Cy3- bzw. Cy5-Farbstoff. Besonders bevorzugt ist hierbei, dass ein Farbstoff entweder an den Antigen-/ Antikörperkomplex bindet, oder in diesen interkaliert.

Weiter bevorzugt ist, dass räumlich getrennt von den Stellen auf der Membran mit den erfindungsgemäßen Antigenen weitere Antigene angebracht sind, die einen spezifischen Nachweis gegen *Chlamydia pneumoniae* ermöglichen. Für Biochip-Anwendungen mit festen Biochip-Oberflächen (Kunststoff) werden besonders bevorzugt die Cy3-, Cy5-Farbstoffe verwendet.

Für das erfindungsgemäße Nachweisverfahren werden biologische Proben verwendet. Diese biologischen Proben enthalten die *Chlamydia trachomatis*-spezifischen Antikörper. Die biologische Probe kann von menschlichen oder tierischen Patienten stammen, bevorzugterweise von menschlichen Patienten. Die biologische Probe kann jede Probe sein, die Körperflüssigkeit oder Gewebe, wie beispielsweise Blut oder Knochenmark enthält. Bevorzugterweise wird Serum oder Plasma verwendet. Zusätzlich bevorzugt ist, dass die Proben einer Zelllyse unterzogen werden, bevor sie mit den Antigenen in Kontakt gebracht werden. Verfahren zur Probenbereitung für Immunoassays sind dem Fachmann bekannt. Die Aufbereitung der Proben kann hierbei beispielsweise das Zentrifugieren, Ausfällen, Aufkonzentrieren, Filtrieren, Dialysieren oder Verdünnen der Probe umfassen. Die Art der Probenaufbereitung ist hierbei von der Technik der Detektion der Antikörper abhängig.

Das erfindungsgemäße Verfahren beruht auf üblichen, dem Fachmann bekannten Techniken der Molekularbiologie, Mikrobiologie, rekombinaten DNA, und Immunologie. Diese Techniken sind in der Literatur ausführlich beschrieben. Diesbezüglich wird insbesondere verwiesen auf: Molecular Cloning, A Laboratory Manual, zweite Ausgabe (1989); DNA Cloning, Volumes I und II (D. N. Glover, Ed., 1985); Oligonucleotide Synthesis (M. J. Gait, Ed, 1984); Nucleic Acid Hybridization (B. D. Hames & S. J. Higgins, Eds., 1984); Transcription And Translation (B. D. Hames & S. J. Higgins, Eds., 1984); Animal Cell Culture (R. I. Freshney, Ed., 1986); lmmobilized Cells And Enzymes (IRL Press, 1986); B. Perbal, A Pracitcal Guide To Molecular Cloning (1984); Die Serien, Methods In Enzymology (Academic Press, Inc.); Gene Transfer Vectors For Mammalian Cells (J. H. Miller und M. P. Calos, Eds., 1987, Cold Spring Harbor Laboratory), Methods in Enzymology Vol. 154 und Vol. 155 (Wu and Grossman, bzw. Wu), Mayer und Walker, Eds. (1987), Immunochemical Methods In Cell And Molecular Biology (Academic Press, London), Scopes, (1987), Protein Purification: Principles And Practice, zweite Ausgabe, (Springer-Verlag, N.Y.), und Handbook Of Experimental lmmunology, Volumes I - IV (D. M. Weir und C. C. Blackwell, Eds, 1986).

Der Nachweis einer *Chlamydia trachomatis -* Infektion erfolgt über den Nachweis von *Chlamydia trachomatis* spezifischen Antikörpern in den Proben durch die erfindungsgemäßen Antigene. Der Begriff "Antikörper" bezeichnet ein immunoglobulin oder ein Derivat davon, das an die erfindungsgemäßen Antigene binden kann.

Die erfindungsgemäßen "Antigene" können an die *Chlamydia trachomatis- spezifischen Antikörper* binden. Die Erfindung betrifft Antigene mit den in den Sequenzprotokollen angegebenen Sequenzen und zusätzlich bevorzugt auch immunogene Fragmente bzw. Teilsequenzen der Aminosäuresequenzen, die in den Sequenzprotokollen angegeben sind und zusätzlich bevorzugt auch immunogene Antigene, die zu den Aminosäuresequenzen aus den Sequenzprotokollen im Wesentlichen gleich sind.

Geeignete Fragmente bzw. Teilsequenzen der Aminosäuresequenzen der Antigene haben üblicherweise eine Länge von mindestens 15 Aminosäuren, bevorzugterweise mindestens 20 Aminosäuren und noch bevorzugter mindestens 25 Aminosäuren. Für die Tests werden bevorzugt solche Fragmente der erfindungsgemäß eingesetzten Antigene verwendet, die wenigstens ein diagnostisch relevantes Epitop aufweisen. Dem Durchschnittsfachmann sind verschiedene Methoden zur Kartierung von Antigenen bekannt. Durch verschiedene Methoden des Epitopmappings kann bestimmt werden, welche Bereiche für die immunologische Diagnostik besonders relevant sind. Lineare Epitope finden sich auf kurzen Aminosäureabschnitten. Konformationsepitope entstehen durch die räumliche Konfiguration des Antigens. Daher sind bevorzugte Fragmente, die Konformationsepitope aufweisen, in der Regel länger, üblicherweise wenigstens 50 Aminosäuren, bevorzugter wenigstens 75 Aminosäuren. Dem Durchschnittsfachmann ist allerdings auch bekannt, dass nur einige wenige Aminosäuren am Epitop beteiligt sind. Andere Aminosäuren, die sich beispielsweise im Inneren des Antigens befinden können verändert sein, ohne dass dies die diagnostische Bedeutung des Epitops verändert.

Der Ausdruck: "Antigene, die zu den Aminosäuresequenzen aus den Sequenzprotokollen im Wesentlichen gleich sind" umfasst hierbei Aminosäuresequenzen bei denen (beispielsweise durch Mutation) 1 bis 20, bevorzugt 1 bis 15, noch bevorzugter, 1 bis 10, besonders bevorzugt 1 bis 5, und am meisten bevorzugt 1 bis 3 Aminosäuren substituiert, deletiert oder addiert wurden im Vergleich zu den Sequenzen aus den Sequenzprotokollen.

Geeignete Fragmente bzw. Teilsequenzen oder "im Wesentlichen" gleiche Antigene sind hierbei Proteine, die eine Selektivität gegenüber *Chlamydia trachomatis*-spezifischen Antikörpern aufweisen. Bevorzugt kommt es bei Durchführung eines erfindungsgemäßen Nachweisverfahrens gemäß den Beispielen bei Verwendung von positive Blutspenderseren ohne klinischem Verdacht auf eine chronische *C.trachomatis*-Infektion bei maximal 3 von 6 Personen, weiter bevorzugt maximal 2 von 6 Personen und noch weiter bevorzugt bei maximal 1 von 6 Personen zu einem falsch-positiven Ergebnis.

Die Antigene werden ausgewählt aus der Gruppe bestehend aus CT017, CT098, CT318-L1P, CT431, CT456-TARP, CT603-TSAP und CT664 oder Fragmenten davon. Weiter bevorzugt sind die Antigenen ausgewählt aus der Gruppe bestehend aus: CT017, CT098, CT318-L1P, CT431, CT603-TSAP und CT664.

In einer bevorzugten Ausführungsform zum Nachweis von chronischen Erkrankungen sind die Antigene besonders bevorzugt ausgewählt aus der Gruppe bestehend aus: CT017, CT098, CT318-L1P, CT431, CT603-TSAP und CT664. Noch weiter bevorzugt für den Nachweis von chronischen Erkrankungen bzw. Infektionen sind die Antigene aus der Gruppe bestehend aus: CT017, CT098, CT318-L1P und CT664. Am meisten bevorzugt sind die Antigene: CT603-TSAP und CT664.

Tabelle 1 zeigt die erfindungsgemäßen Antigene mit ihren Sequenznummern (SEQ ID NO), wobei die Sequenzen im Sequenzprotokoll wiedergegeben sind.

Bevorzugt werden mindestens ein, weiter bevorzugt mindestens zwei, noch weiter bevorzugt mindestens vier, und am meisten bevorzugt mindestens sechs Antigene aus den vorstehend genannten Gruppen von Antigenen ausgewählt. In einer besonders bevorzugten Ausführungsform wird die gesamte Gruppe der *Chlamydia trachomatis -* spezifischen Antigenen für das Nachweisverfahren verwendet.

Zusätzlich zu den oben angegebenen Antigenen können auch weitere *Chlamydia trachomatis -* spezifische Antigene in dem erfindungsgemäßen Nachweisverfahren verwendet werden. Falls die Detektion der Antigen-/Antikörperkomplexe selektiv erfolgt, können zusätzlich und gleichzeitig auch andere Antigene gegen andere Erreger in dem gleichen Nachweisverfahren verwendet werden. Beispielsweise könnten die Antigen-/Antikörperkomplexe der verschiedenen Erreger aufgrund unterschiedlicher spektroskopischer Eigenschaften nebeneinander analysiert werden.

**Tabelle 1. Chlamydia trachomatis-Antigene (Bei den Genbanknummern handelt es sich um die Nummern, unter denen die Gesamtgenome zweier Isolate von Chlamydia trachomatis zu finden sind. Einen separaten Eintrag für die einzelnen Gene gibt es bisher nicht. Diese sind anhand der Nummer des offenen Leserahmens mit vorgestelltem CT für Chlamydia trachomatis im Gesamtgenom unterschieden; Maßgeblich sind jedoch die Sequenzprotokolle):**

| Antigen | SEQ ID NO | Genbank-Nummer |
|---|---|---|
| CT017 | 1 | NC000117 |
| CT098 | 2 | NC000117 |
| CT318-L1P | 3 | CP000051 |
| CT431 | 4 | NC000117 |
| CT456-TARP | 5 | NC000117 |
| C/603-TSAP | 6 | NC000117 |
| CT664 | 7 | NC000117 |

Die Herstellung der Antigene kann durch bekannte Verfahren erfolgen. Insbesondere können die Antigene in bakteriellen Expressionsvektoren kloniert und rekombinant exprimiert werden. Hierzu geeignete Organismen sind dem Fachmann bekannt und umfassen beispielsweise das Bakterium *Escherichia coli.* Die Herstellung der Antigene ist in Beispiel 1 beschrieben.

Der Nachweis der Antikörper kann bevorzugt durch Immunoassays erfolgen. Hierbei können entweder direkte oder indirekte Immunoassays verwendet werden. Diese Assays umfassen, sind jedoch nicht darauf beschränkt: kompetitive Bindungsassays, nicht-kompetitive Bindungsassays, Radioimmunoassays, Enzyme-Linked Immunosorbent-Assays (ELISA), Western-Blot, Line-Assay, Sandwich-Assays, Fällungsreaktionen, Gel-Diffusion-Immunodiffusionsassays, Agglutinationsassays, Fluoreszenzimmunoassays, Chemolumineszenzimmunoassays, ImmunoPCR-Immunoassays, Protein A oder Protein G Immunoassays und Immunoelektrophoreseassays.

Besonders vorteilhaft ist die Verwendung eines Enzyme-Linked Immunosorbent Assays (ELISA), Western-Blots oder Line-Assays, weiter bevorzugt ELISA. Diese Verfahren basieren auf Antigen-Antikörper-Wechselwirkungen, wobei der entstehende Antigen-/Antikörperkomplex durch bekannte Nachweisverfahren detektiert werden kann. Besonders bevorzugt ist hierbei die Verwendung von ELISA-Verfahren, Western-Blots und Line-Assays mit peroxidasekonjugierten Sekundärantikörpern, wobei der Nachweis der Antigen-Antikörper-Reaktion über ein Färbesubstrat (TMB) erfolgt.

Insbesondere geeignet sind ELISA-Testverfahren, in denen die Antigene an eine feste Phase meist nicht-kovalent (hydrophobe Wechselwirkungen) gebunden sind und mit einer Probenflüssigkeit in Kontakt gebracht werden. Als feste Phase kann beispielsweise eine Mikrotiterplatte verwendet werden. Das Protein (Antigen) kann dann mit der festen Phase bzw. der Oberfläche der Mikrotiterplatte, üblicherweise aus Polystyrol, z.B. bei hohem pH-Wert wechselwirken. Bevorzugterweise wird ein Biochip als feste Phase verwendet.

Indikatorsysteme für den Nachweis der Antigen-/Antikörperkomplexe sind dem Fachmann bekannt. Beispielsweise kann nach Bildung des Antigen-/Antikörperkomplexes ein zweiter Antikörper verwendet werden, der den konstanten Teil des ersten Antikörpers erkennt oder auf andere Weise spezifisch an den Antigen-/Antikörperkomplex bindet. Der zweite Antikörper bzw. die Verbindungen zur Detektion des Antigen-/Antikörperkomplexes, beispielsweise ein monoklonaler oder polyklonaler Antikörper oder ein Fragment davon, besitzt bevorzugterweise eine Markierung. Noch bevorzugter weist er eine nicht-radioaktive Markierung wie beispielsweise eine Enzym-Markierung, Fluoreszenz-Markierung, Lichtemissions-Markierung und so weiter auf. Besonders bevorzugt wird eine Enzym-Markierung wie eine alkalische Phosphatase, β-Galactosidase oder Meerretichperoxidase auf.

Der zweite Antikörper kann beispielsweise an eine alkalische Phosphatase gekoppelt sein, wobei die alkalische Phosphatase dann eine enzymatische Chromogenumwandlung katalysiert. Das farblose Chromogen wird dann zu einem Farbstoff umgesetzt und vermessen. Die Menge an freigesetztem Farbstoff korreliert dann mit der Menge des gesuchten Antikörpers. Weitere kompetitive oder nicht-kompetitive Messverfahren sind dem Fachmann bekannt.

Des Weiteren können auch Verbindungen oder Marker zur direkten Detektion des Antigen-/Antikörperkomplexes verwendet werden, so wie beispielsweise radioaktive, fluoreszierende biologische oder enzymatische Tags. Besonders vorteilhaft ist es, wenn der Antigen-/Antikörperkomplex mit dem Farbstoff verbunden ist und die Stelle auf der Membran detektiert werden kann, auf der der Antigen-/ Antikörperkomplex mit den erfindungsgemäßen Antigenen aufgebracht ist. Dies ermöglicht den selektiven, gleichzeitigen Nachweis von verschiedenen Krankheitserregern, beispielsweise *Chlamydia trachomatis oder Chlamydia pneumoniae.* Bevorzugt können dann die Testergebnisse optisch ausgewertet werden, wobei beispielsweise farbige Stellen den positiven Nachweis der Krankheitserreger anzeigen, deren Antigene an die Stelle angebracht wurden. Des Weiteren ist bevorzugt, dass sich die optisch detektierbaren Antigen-/ Antikörperkomplexe farblich bzw. in ihrem Wellenlängenabsorptionsverhalten unterscheiden. So können die Antigen-/ Antikörperkomplexe zusätzlich zu oder anstelle von einer räumlichen Kennzeichnung noch durch ihr Absorptionsverhalten charakterisiert werden. Die Verwendung von peroxidasekonjugierten Sekundärantikörpern, wobei der Nachweis der Antigen-Antikörper-Reaktion über ein Färbesubstrat (TMB) erfolgt, bzw. die Verwendung von Fluoreszenzfarbstoffen wie Cy3 bzw. Cy5 ist bevorzugt. Alle Verfahren zur Detektion des Antigen-/Antikörperkomplexes können sowohl für die Verfahren mit als auch ohne Biochip bzw. festen Träger eingesetzt werden.

Eine weitere Ausführungsform der Erfindung umfasst ein Test-Kit zum selektiven Nachweis von *Chlamydia trachomatis*-Infektionen. Das Test-Kit umfasst die oben angegebenen *Chlamydia trachomatis*-spezifischen Antigene. Die an anderer Stelle beschriebene Anzahl an verschiedenen *Chlamydia trachomatis*-spezifischen Antigenen ist ebenfalls für das Test-Kit geeignet. Die Durchführung des Verfahrens und die Techniken zur Detektion der Antigen-/Antikörperkomplexe sind auf das Test-Kit anwendbar. Insbesondere kann das Test-Kit zusätzlich die zur Detektion der Antigen-/Antikörperkomplexe notwendigen Substanzen enthalten.

Des Weiteren kann das Test-Kit zusätzlich andere Komponenten wie Waschpuffer oder Zusammensetzungen, die einen *Chlamydia trachomatis*-spezifischen Antikörper als Standard enthalten, umfassen. Zusätzlich kann das Test-Kit auch eine Mikrotiterplatte oder einen Biochip umfassen, auf dem die Antikörper immobilisiert sind. Des Weiteren kann das Test-Kit auch Informationsmaterialien wie beispielsweise eine Gebrauchsanweisung enthalten, die die Probenbereitung und/oder die Vorschrift zur Durchführung des Assays beschreiben.

Eine Ausführungsform der Erfindung umfasst einen Biochip, auf dem die erfindungsgemäßen Antigene aufgebracht sind. Die an anderer Stelle beschriebene Anzahl an verschiedenen *Chlamydia trachomatis*-spezifischen Antigenen ist ebenfalls für das Verfahren mit einem Biochip geeignet. Die Durchführung des Verfahrens und die Techniken zur Detektion der Antigen-/Antikörperkomplexe sind auch auf das Verfahren mit einem Biochip anwendbar. Insbesondere können die Antigene auf der Membran des Biochips angebracht werden.

Für die Membran können beispielsweise poröse, natürlich vorkommende oder synthetische Polymere, Glas, keramische Materialien, Zellulosematerialien oder Ähnliches verwendet werden. Die Materialien könne beispielsweise aus Filmen, Fasern oder Partikeln (beispielsweise Kügelchen, die durch Kleb- oder Bindematerialien zusammengehalten werden) zu einer Membran gefertigt werden. Besonders bevorzugt besteht die Membran aus Polycarbonat, Nitrozellulose, Zelluloseacetat, Polyamid, Polyester, Polyvinylidenfluorid oder Nylon. Am meisten bevorzugt besteht die Membran aus Nitrozellulose. Der Ausdruck "Nitrozellulose" bezieht sich auf Esterzellulose mit Nitrat-Gruppierungen. Der Ausdruck "Nitrozellulose" umfasst zudem Esterzellulose mit Nitrat-Gruppierungen alleine oder mit einem Gemisch aus verschiedenen Estern, die durch andere Säuren als Nitriersäure erzeugt wurden, insbesondere aliphatische carbozyklische Säuren. Der Ausdruck "aufgebracht" bedeutet, dass die Antigene eine ausreichende Affinität zu der Membran aufweisen oder sogar chemisch mit dieser verbunden sind, so dass das Aufbringen der Probenflüssigkeit bzw. der eventuell weiter notwendigen Wasch- und Reaktionslösungen nicht zur Ablösung dieser Antigene führt.

Das erfindungsgemäße Verfahren kann wie folgt durchgeführt werden (siehe auch Beispiel 2 und 3):

Der erste Schritt der serologischen Nachweisreaktion ist die Immobilisierung der Antigene auf dem festen Träger bzw. der festen Phase, beispielsweise der Nitrozellulosemembran, der Mikrotiterplatte oder dem Biochip. Hierbei werden die erfindungsgemäßen Antigene oder eine Auswahl aus der Gruppe der erfindungsgemäßen Antigene, beispielsweise durch hydrophobe Wechselwirkungen an der Oberfläche immobilisiert.

Nach einem eventuellen Waschschritt werden die immobilisierten Antigene dann mit der Probenflüssigkeit in Kontakt gebracht. Die Aufarbeitung der Proben kann nach den oben beschriebenen Verfahren und wie in den Beispielen erläutert erfolgen, ist jedoch nicht darauf beschränkt. Durch die Antigen-/ Antikörper-Wechselwirkung werden die *Chlamydia trachomatis*-spezifischen Antikörper aus der Probenflüssigkeit an die erfindungsgemäßen Antigene gebunden. Es bilden sich Antigen-/ Antikörperkomplexe.

Nach einer geeigneten Inkubationszeit wird der feste Träger gewaschen und von den Rückständen der Probe befreit. Anschließend wird eine Lösung zum Detektieren des Antigen-/Antikörperkomplexes mit den Antigen-/ Antikörperkomplexen in Kontakt gebracht, wobei eine quantitative oder qualitative Detektion beispielsweise spektroskopisch erfolgen kann. Hierbei kann beispielsweise eine automatisierte Messung der Farbtiefe des Chromogens durchgeführt werden.

In einer Ausführungsform der Erfindung wird der Krankheitsverlauf der *Chlamydia trachomatis*-Infektion ausgewertet. Für diese Ausführungsform des Verfahrens kann auch das erfindungsgemäße Test-Kit oder der erfindungsgemäße Biochip verwendet werden. Hierbei werden mindestens zwei, weiter bevorzugt mindestens drei, noch weiter bevorzugt mindestens vier und am meisten bevorzugt mindestens fünf Proben des gleichen Patienten zu unterschiedlichen Zeitpunkten getestet und die Anwesenheit von *Chlamydia trachomatis* bzw. die Menge der *Chlamydia trachomatis*-spezifischen Antikörper gemessen. Dieses Verfahren kann das Sammeln von Daten über einen bestimmten Zeitraum einschließen. Die Proben der Patienten können zu regelmäßigen oder unregelmäßigen, bevorzugterweise regelmäßigen Zeitpunkten entnommen werden. Der Abstand zwischen der Entnahme von zwei Proben kann zwischen einer Woche und zwölf Monaten, bevorzugterweise zwischen zwei Wochen und vier Monaten und weiter bevorzugt zwischen 4 und 6 Wochen liegen, um den Titeranstieg zu verfolgen. Dieses Verfahren erlaubt den Fortschritt der *Chlamydia trachomatis*-Infektion zu beobachten.

### Beispiel 1 - Identifizierung der erfindungsgemäßen Antigene

Die Identifizierung und Charakterisierung der *Chlamydia trachomatis*-spezifischen -Antigene wurde mittels der 2D-Gelelektrophorese und Massenspektrometrie durchgeführt.

Dazu wurde zunächst eine induzierbare Genexpressionsdatenbank für *Chlamydia pneumoniae* und *Chlamydia trachomatis* in *Escherichia coli* hergestellt. Dazu wurden Seren von Patienten mit klinisch und mikrobiologisch gesicherten *Chlamydia pneumoniae* bzw. *Chlamydia trachomatis-*Infektionen gepoolt und gegen in-vitro exprimierte Chlamydien-Antigene präabsorbiert. Dazu wurden Lysate von *Chlamydia pneumoniae*-infizierten HeLa229-Zellen zu verschiedenen Infektionszeitpunkten präpariert.

Nach Kolonieplot-Analyse mit den so präabsorbierten Seren werden die reaktiven Klone isoliert und die klonierten DNA-Fragmente sequenziert. Durch Vergleich mit den bereits annotierten Sequenzen von *Chlamydia trachomatis* und *Chlamydia pneumoniae* konnte die Identität der in-vivo exprimierten Antigene geklärt werden.

Im Detail erfolgte die Identifizierung der Antigene wie folgt: *Chlamydia trachomatis* serovar D/ UW-3/Cx wurde auf HeLa 229 kultiviert; 48-72h nach Infektion wurden die Zellen geerntet und homogenisiert. *Chlamydia trachomatis* wurde durch nachfolgende Zentrifugationsschritte vom Zellrückständen isoliert. Die erhaltenen Elementarkörperchen von *Chlamydia trachomatis* wurden mit Detergenzien lysiert und mit Ultraschall behandelt. Der erste Schritt der 2D-Immunelektrophorese wurde mit Ready IPG Strips, pH 3-10, 11 cm (BioRad); anschließend wurde eine SDS-PAGE in einem 10% Polyacrylamidgel durchgeführt. Die Gele wurden für Western-Blots geblottet bzw. für die nanoLC-ESI-MSMS-Analyse mit Commassie Brilliant Blue gefärbt. Die Western-Blots erfolgten mit Seren von Patienten mit einem klinischen Bild einer chronischen Infektion mit *Chlamydia trachomatis* in einer Verdünnung von 1:125. Reaktive Spots wurden aus den gefärbten und getrockneten Gelen der 2D-Immunelektrophorese ausgeschnitten, und mit der nanoLC-ESI-MSMS Methode analysiert. Dabei wurden die Proteine in den Spots mit Trypsin verdaut, entsalzt und aufkonzentriert. Die nanoLC-ESI-MSMS-Analyse wurde mit dem Esquire 3000 plus-Gerät (Bruker Daltronics) durchgeführt Die Proteine wurden mittels MS/MS ion search (Matrix Science) identifiziert und die Sequenzen mit öffentlich zugänglichen Datenbanken verglichen.

Die genomischen Sequenzen aller Antigene wurden mittels PCR und spezifischen Oligodesoxynukleotiden als Primern aus der genomischen DNA von *Chlamydia trachomatis* amplifiziert und die Amplifikate gereinigt. Die gereinigten Amplifikate wurden mit geeigneten Restriktionsendonukleasen, deren Spaltsequenzen in den PCR-Primern enthalten waren, gespalten, mittels Agarosegelelektrophorese gereinigt und in die Vektoren pUC8 bzw. pDS1, die mit denselben Restriktionsendonukleasen gespalten worden waren, ligiert. Kompetente *Escherichia coli* wurden mit den Ligationsprodukten transformiert. Positive Klone wurden isoliert und wurden mittels Restriktionsanalyse und DNA-Sequenzierung charakterisiert. Die Expression der Antigene erfolgte in *Escherichia coli.* Die Expression der Antigene wurde in der SDS-PAGE mit Färbung durch Coomassie Brilliant Blue sowie im Western-Blot mit spezifischen humanen Seren von Patienten mit Antikörpern gegen *Chlamydia trachomatis* nachgewiesen. Die Antigene wurden anschließend mit chromatographischen Methoden gereinigt.

Tabelle 2 zeigt die Klondaten der *Chlamydia trachomatis*-Antigene.

**Tabelle 2. Klondaten für Chlamydia trachomatis-Antigene:**

| Antigen | Vektor | RE | Einschub [bp] | theoreti sch [kDa] | Expression [kDa] | Expression [Menge] |
|---|---|---|---|---|---|---|
| CT017 | pDS1 | *BamHI-Pstl* | 58-1299 | 46 | 48 | +++ |
| CT098 | pDS1 | *Sall-Pstl* | 1-1707 | 64 | 40+36 | ++ |
| CT318-L1P | pDS1 | *BamHI-Sall* | 1-696 | 26 | 24 | +++ |
| CT431 | pUC8 | *BamHI-Pstl* | 1-534 | 20 | 21 | ++ |
| CT456-TARP | pDS1 | *BamHI-Sall* | 1-3015 | 113 | >100 | ++ |
| CT603-TSAP | pDS1 | *BamHI-Pstl* | 1-585 | 22 | 23 | +++ |
| CT664 | pDS1 | *BamHI-Xho*/*SaII** | 1-2487 | 93 | ~100 | + |

Die Seroreaktivität der rekombinanten exprimierten Proteine wurde mit denselben spezifischen Seren, die zur Identifizierung eingesetzt wurden, überprüft.

Für einzelne rekombinante Proteine wurde eine Epitopcharakterisierung durchgeführt, um spezies-spezifische Epitope zu identifizieren und abzugrenzen. Dies erfolgte durch Subklonierung einzelner Teilfragmente und Analyse der Seroreaktivität im Western-blot bzw. mittels in Festphasensynthese hergestellter Peptide und Analyse der Seroreaktivität im Line-Assay in Abhängigkeit von Sequenzhomologien. Die Seroreaktivität der identifzierten Antigene und spezies-spezifischen Teilfragmente bzw. Peptide wurde daraufhin im Line-Assay analysiert, um eine optimale Kombination der rekombinanten Antigene zu finden, die einerseits eine genaue Spezies-Differenzierung, andererseits eine Unterscheidung zwischen aktiven und abgelaufenen Chlamydien-Infektionen ermöglicht.

Um nachzuweisen, dass humane Antikörper gegen die Antigene CT017, CT098, CT318-L1P, CT431, CT456-TARP, CT603-TSAP und CT664, insbesondere bei chronischen Infektionen mit *Chlamydia trachomatis* auftreten, wurden die Seren von 24 Patienten mit klinischem Verdacht auf eine chronische Infektion mit *Chlamydia trachomatis,* sowie 6 Seren von gesunden Blutspendern mit Antikörpern gegen *Chlamydia trachomatis,* aber ohne Verdacht auf eine chronische Infektion mit *Chlamydia trachomatis,* verglichen. Die Ergebnisse sind in Tabelle 3 zusammengefasst.

Dabei zeigt sich, dass die Antigene CT017, CT098, CT318-L1P, CT431, CT603-TSAP und CT664 insbesondere mit Seren von Patienten mit klinischem Verdacht auf eine chronische Infektion mit *Chlamydia trachomatis* reagieren, aber kaum mit den Seren der gesunden Blutspendern mit Antikörpern gegen *Chlamydia trachomatis,* aber ohne Verdacht auf eine chronische Infektion. Das Antigen CT456-TARP reagierte mit beiden Gruppen in vergleichbarer Häufigkeit. Im Gegensatz dazu sind die bereits bekannten Antigene CT858-CPAF, CT089, CT795 und CT813 weniger dazu geeignet, zwischen Patienten mit klinischem Verdacht auf eine chronische Infektion mit *Chlamydia trachomatis* sowie gesunden Blutspendern mit Antikörpern gegen *Chlamydia trachomatis,* aber ohne Verdacht auf eine chronische Infektion mit *Chlamydia trachomatis,* zu unterscheiden.

Außerdem sind die Antigene CT098, CT431, CT603-TSAP und CT664 deutlich spezifischer als die bereits bekannten Antigene CT858-CPAF, CT089, CT795 und CT813 für eine Infektion mit *Chlamydia trachomatis* gegenüber einer Infektion mit *Chlamydia pneumoniae.* So reagierten die Antigene CT098, CT431, CT603-TSAP und CT664 mit keinem der 20 Seren von Patienten mit Antikörpern gegen *Chlamydia pneumoniae,* während die Antigene CT858-CPAF, CT089, CT795 und CT813 mit jeweils einem oder zwei Seren der 20 Seren von Patienten mit Antikörpern gegen *Chlamydia pneumoniae* reagierten.

**Tabelle 3. Serologische Charakterisierung der erfindungsgemäßen Chlamydia trachomatis-Antigene. Die bereits bekannten, nicht erfindungsgemäßen Antigene sind mit einem "*" markiert.**

| **Antigen** | ***C.pneumoniae*-positive Seren ohne Verdacht auf eine *C.trachomatis-*Infektion n = 20** | **Seren von Patienten mit klinischem Verdacht auf eine chronische *C.trachomatis*-Infektion n = 24** | ***C.trachomats*-positive Blutspenderseren ohne klinischem Verdacht auf eine chronische *C.trachomatis*-Infektion n=6** |
|---|---|---|---|
| CT017 | 4 | 11 | 1 |
| CT098 | 1 | 16 | 1 |
| CT318 | 4 | 14 | 1 |
| CT431 | 0 | 7 | 1 |
| CT456 | 3 | 22 | 5 |
| CT603 | 0 | 6 | 0 |
| CT664 | 0 | 12 | 0 |
| | | | |
| CT089 * | 3 | 17 | 2 |
| CT795 * | 2 | 21 | 3 |
| CT813 * | 7 | 23 | 6 |
| CT858 * | 1 | 9 | 2 |

### Beispiel 2 - Durchführung des Testverfahrens

Die Expression der Antigene erfolgte in *Escherichia coli,* angezogen in Schüttelkolben oder in einem Fermenter. Die Bakterien wurden durch Zentrifugation geerntet und mittels Detergentien und Ultraschallbehandlung lysiert. Die Antigene lagen danach zum Teil in "inclusion bodies" oder löslich vor. Die in "inclusion bodies" vorliegenden Antigene wurden mit Harnstoff solubilisiert.

Anschließend wurden die Antigene mit chromatographischen Methoden, insbesondere Anionen- und Kationenaustausch, schrittweise gereinigt. Die gereinigten Antigene wurden in empirisch gefundenen Verdünnungen auf ein Polyacrylamidgel aufgetragen und im elektrischen Feld der Größe nach aufgetrennt. Nach der Gelelektrophorese wurden die Antigene im elektrischen Feld auf eine Nitrozellulosemembran geblottet und damit auf der Membran immobilisiert.

Das Aufbringen auf die Membran kann jedoch auch direkte durch Aufsprühen auf die Nitrozellulosemembran von empirisch ermittelten Verdünnungen der gereinigten Antigene erfolgen. Die Membranen wurden nach dem Blot-Transfer mit einer proteinhaltigen Lösung behandelt, um noch nicht besetzte Proteinbindungsstellen auf der Nitrozellulosemembran abzusättigen.

Die Membranen wurden anschließend getrocknet und in Streifen geschnitten. Je ein Streifen wurde anschließend mit einem humanen Serum in einer Verdünnung von 1:250 über Nacht bei Raumtemperatur unter leichtem Schütteln inkubiert. Dabei binden die Antikörper in dem Patientenserum an die auf der Nitrozellulosemembran immobilisierten Antigene.

Die Nitrozellulosemembran-Streifen wurden anschließend 3 × gewaschen und nachfolgend für 60 min mit einem Sekundärantikörper inkubiert, der mit Meerrettich-Perixidase konjugiert war.

Anschließend wurden die Nitrozellulosemembran-Streifen erneut 3 × gewaschen und in Folge mit einer TMB(Tetramethylbenzidin)-Lösung behandelt. An den Stellen, an denen Antigene immobilisiert waren, die mit Patientenantikörpern reagiert hatten, entstand daraufhin ein Farbniederschlag auf den Nitrozellulosemembran-Streifen. Die Streifen wurden anschließend getrocknet und die Färbungen analysiert (Tabelle 3).

### Beispiel 3 - Herstellung eines Biochips

Die Antigene wurden durch ein kontaktloses Dispensierverfahren AD3050, Biodot) auf einen ca. 1 cm² großen Kunststoffchip aufgebracht, der z.B. aus Polycarbonat oder Polystyrol mit entsprechend aktivierter Oberfläche besteht. Der Abstand zwischen den einzelnen Antigendots beträgt 1,125 mm. Insgesamt können bis zu 99 Antigene auf den Biochip aufgetragen werden. Als Antigene werden die Antigene CT017, CT098, CT318-L1P, CT431, CT456-TARP, CT603-TSAP und CT664 von *Chlamydia trachomatis* auf den Biochip aufgebracht. Zusätzlich können Antigene, z.B. von *Chlamydia pneumoniae* und *Chlamydia psittaci* auf den Biochip aufgebracht werden, um den Status humanpathogener Chlamydieninfektionen abzuklären.

Außerdem können zusätzlich z.B. noch Antigene der Bakterien *Yersinia enterocolitica, Salmonella Enteritidis, Salmonella Typhimurium, Campylobacter jejuni, Chlamydia trachomatis, Chlamydia pneumoniae, Borrelia burgdorferi, Streptococcus pyogenes,* der Viren Parvovirus B19 und Epstein-Barr-Virus sowie von humanen Autoimmunantigenen aufgetragen werden, um reaktive bzw. infektabhängige Arthritis zu diagnostizieren und von Autoimmunerkrankungen abzugrenzen. Außerdem können für weitere Fragestellungen andere Antigene oder Kombinationen von diesen mit anderen Antigenen auf den Biochip aufgetragen werden.

Der Biochip wird in eine Kunststoffkartusche eingesetzt. Die Kunststoffkartusche bildet ein abgeschlossenes System mit den Komponenten: Kartuschenkörper mit Serumbehälter und Mikrofluidkanälen, Kunststoffchip mit immobilisierten Antigenen, Deckfolie zum Versiegeln der Kartusche und elastisches Septum für die fluidische Ankopplung an einen Assayprozessor, der die erforderlichen Reagenzlösungen wie Puffer, Fluoreszenzfarbstoffkonjugate und Konditioniermedien in die BioChip-Kartusche pumpt.

Im ersten Schritt der Testablaufes wird der Serumbehälter der Kartusche mit verdünntem Serum befüllt und die Kartusche mit dem integriertem Biochip in den Assayprozessor eingesetzt und das Serum gleichmäßig über das Dot-Array gesaugt. Nach der Seruminkubation erfolgt die Reaktion gebundener Antikörper mit Fluoreszenzfarbstoffkonjugaten. Sowohl im Anschluss an die Serum- als auch an die Konjugatinkubation sorgt ein Spülzyklus für die Entfernung der nicht gebundenen Serum- bzw. Konjugatreste. Die Fluoreszenz wird anschließend quantitativ in einem Fluoreszenzreader fluoreszenzspektrometrisch ausgewertet. Durch die Verwendung von Farbstoffkonjugaten mit unterschiedlichen Immunglobulinen, z.B. Anti-human-IgG und Anti-human-IgM, können unterschiedliche Immunglobulinklassen, die in unterschiedlichen Stadien der Infektion in unterschiedlichen Mengen vorliegen quantitativ nachgewiesen werden.

### Beispiel 4 - Durchführung des Testverfahrens mit einem Biochip

Das erfindungsgemäße Testverfahren kann auch unter Verwendung eines Biochips durchgeführt werden. Die Durchführung des Verfahrens erfolgt analog zu Beispiel 2, wobei die Immobilisierung der Antigene auf einem Biochip erfolgt.

Dabei kann die Oberfläche des Biochips, auf der die Antigene immobilisiert werden, aus einer Nitrozellulosemembran, aber auch aus aktiviertem Kunststoff (beispielsweise Polystyrol, Polycarbonat, Keramik) oder aktiviertem Glas bestehen. Die Antigene können mit einem Dispensiergerät, z.B. BioDot AD3050, automatisch direkt auf die Oberfläche des Biochips aufgesprüht werden.

Nach der Immobilisierung der Antigene auf dem Biochip werden die Biochips mit einer proteinhaltigen Lösung behandelt, um noch nicht besetzte Proteinbindungsstellen auf der Oberfläche des Biochips abzusättigen. Das weitere Testverfahren erfolgt analog der Prozessierung der Nitrozellulosemembran-Streifen, wie in Beispiel 2 beschrieben.

Der Nachweis der Antigen-Antikörper-Reaktion kann zusätzlich zur Färbung mit einem peroxidasemarkierten Sekundärantikörper und dem Farbsubstrat TMB auch mit einem Sekundärantikörper erfolgen, an den ein Fluoreszenzfarbstoff konjugiert ist. Die Fluoreszenz kann dann in einem Fluoreszenzscanner, z.B. Tecan LS 200, bestimmt werden.

## Patentansprüche

1. Verfahren zum selektiven Nachweis von *Chlamydia trachomatis* Infektionen, **dadurch gekennzeichnet, dass** Antigene zum Nachweis von Antikörpern in Proben verwendet werden, wobei die Antigene ausgewählt sind aus der Gruppe bestehend aus: CT017, CT098, CT318-L1P, CT431, CT456-TARP, CT603-TSAPund CT664, sowie aus Fragmenten und Teilsequenzen der vorstehenden Antigene, wobei die Fragmente wenigstens 15 aufeinanderfolgende Aminosäuren der Antigene aufweisen und aus Antigenen, die zu den vorstehenden Antigenen im Wesentlichen gleich sind.

2. Verfahren nach Anspruch 1, **dadurch gekennzeichnet, dass** mindestens zwei Antigene für das Verfahren verwendet werden.

3. Verfahren nach einem der vorangegangenen Ansprüche, **dadurch gekennzeichnet, dass** das Verfahren zur Detektion der Antikörper den Nachweis eines Antigen-/Antikörperkomplexes umfasst.

4. Verfahren nach einem der vorangegangenen Ansprüche, **dadurch gekennzeichnet, dass** das Verfahren zur Detektion der Antikörper mittels eines ELISA (Enzyme-Linked Immunosorbent Assay) Testverfahrens, Western Blots oder Line-Assays erfolgt.

5. Verfahren nach einem der vorangegangenen Ansprüche, **dadurch gekennzeichnet, dass**, die Fragmente oder Teilsequenzen der Antigene eine Länge von mindestens 20 Aminosäuren aufweisen und die im Wesentlichen gleichen Antigene im Vergleich zu den Aminosäuresequenzen aus den Sequenzprotokollen 1 bis 20 Aminosäuren aufweisen die substituiert, addiert oder deletiert sind.

6. Verfahren zum Analysieren des Krankheitsverlaufs einer *Chlamydia trachomatis -* Infektion, **dadurch gekennzeichnet, dass** in bestimmten Zeitabständen Proben von einem Patienten genommen werden und die Proben nach dem Verfahren gemäß der vorangegangenen Ansprüche untersucht werden.

7. Verfahren nach einem der vorangegangenen Ansprüche, **dadurch gekennzeichnet, dass** die zu untersuchenden Proben einer Zelllyse unterzogen werden, bevor sie mit den Antigenen in Kontakt gebracht werden.

8. Test-Kit zum selektiven Nachweis von *Chlamydia trachomatis -* Infektionen, **dadurch gekennzeichnet, dass** das Test-Kit Antigene enthält, ausgewählt aus der Gruppe bestehend aus: CT017, CT098, CT318-L1 P, CT431, CT456-TARP, CT603-TSAPund CT664.

9. Biochip, **dadurch gekennzeichnet, dass** er Antigene, ausgewählt aus der Gruppe bestehend aus: CT017, CT098, CT318-L1P, CT431, CT456-TARP, CT603-TSAP und CT664, für den Nachweis von *Chlamydia trachomatis* -spezifischen Antikörpern aufweist.

10. Biochip nach Anspruch 6, **dadurch gekennzeichnet, dass** er mindestens zwei *Chlamydia trachomatis* -spezifische Antigene aufweist.

11. Biochip nach Anspruch 6 oder 7, **dadurch gekennzeichnet, dass** die *Chlamydia trachomatis*-spezifischen Antigene auf einer Stelle der Membran des Biochips angebracht sind, die räumlich getrennt ist von anderen Stellen auf der Membran, auf der andere Antigene angebracht sind.

12. Biochip nach einem der Ansprüche, **dadurch gekennzeichnet, dass** räumlich getrennt von den *Chlamydia trachomatis*-spezifischen Antigenen weitere Antigene auf der Membran angebracht sind, die einen spezifischen Nachweis gegen *Chlamydia pneumoniae* oder *Chlamydia psittaci* ermöglichen.


SEQUENCE LISTING

<110>  MIKROGEN molekularbiologische Entwicklungs-GmbH

<120>  Biochip und Verfahren zum selektiven Nachweis von Chlamydia
       trachomatis-Infektionen

<130>  MIKROGEN

<160>  7

<170>  PatentIn version 3.3

<210>  1
<211>  433
<212>  PRT
<213>  Chlamydia trachomatis

<400>  1

Met Leu Ile Phe Ala Leu Ser Phe Gly Ala Asp Ala Cys Leu Cys Ala
1               5                   10                  15

Ala Asp Leu Ser Lys Ala Lys Val Glu Ala Ser Val Gly Asp Arg Ala
            20                  25                  30

Ala Phe Ser Pro Phe Thr Gly Glu Ile Lys Gly Asn Arg Val Arg Leu
        35                  40                  45

Arg Leu Ala Pro His Thr Asp Ser Phe Ile Ile Lys Glu Leu Ser Lys
    50                  55                  60

Gly Asp Cys Leu Ala Val Leu Gly Glu Ser Lys Asp Tyr Tyr Val Val
65                  70                  75                  80

Ala Ala Pro Glu Gly Val Arg Gly Tyr Val Phe Arg Thr Phe Val Leu
                85                  90                  95

Asp Asn Val Ile Glu Gly Glu Lys Val Asn Val Arg Leu Glu Pro Ser
            100                 105                 110

Thr Ser Ala Pro Ile Leu Ala Arg Leu Ser Lys Gly Thr Val Val Lys
        115                 120                 125

Thr Leu Gly Ala Ala Gln Gly Lys Trp Ile Glu Ile Ala Leu Pro Lys
    130                 135                 140

Gln Cys Val Phe Tyr Val Ala Lys Asn Phe Val Lys Asn Val Gly Ala
145                 150                 155                 160

Leu Asp Leu Tyr Asn Gln Lys Glu Gly Gln Lys Lys Leu Ala Leu Asp
                165                 170                 175

Leu Leu Ser Ser Ala Met Asp Phe Ala Asp Ala Glu Leu Gln Lys Lys
            180                 185                 190

Ile Glu Asp Ile Asp Leu Asp Ala Ile Tyr Lys Lys Met Asn Leu Ala
        195                 200                 205

Gln Ser Glu Glu Phe Lys Asp Val Pro Gly Leu Gln Ser Leu Val Gln
    210                 215                 220

Lys Ala Leu Glu Arg Val Gln Glu Ala Phe Leu Ala Lys Ser Leu Glu
225                 230                 235                 240

Lys Ser Ser Val Lys Val Pro Glu Ile Arg His Lys Val Leu Glu Glu
                245                 250                 255

Ile Ala Val Val Ser Pro Ala Val Glu Glu Thr Pro Val Val Thr Lys
            260                 265                 270

Thr Glu Glu Gln Lys Val Thr Thr Val Pro Val Pro Ala Pro Ala Val
        275                 280                 285

Val Thr Glu Pro Ala Gln Asp Leu Ser Ser Val Lys Gly Ser Leu Leu
    290                 295                 300

Ser His Tyr Ile Arg Lys Lys Gly Phe Val Lys Ala Ser Pro Val Ile
305                 310                 315                 320

Glu Gly Arg Glu Ser Phe Glu Arg Ser Leu Phe Ala Val Trp Leu Ser
                325                 330                 335

Leu Gln Pro Glu Glu Ile Arg His Gln Leu Thr Met Glu Ser Phe Tyr
            340                 345                 350

Arg Asp Glu Gln Lys Lys Lys Arg Val Leu Thr Gly Glu Leu Glu Val
        355                 360                 365

Tyr Pro His Ile Val Lys Asn Asn Pro Gly Asp Tyr Leu Leu Lys Asn
    370                 375                 380

Gly Glu Asp Val Val Ala Phe Val Tyr Ala Thr Ser Ile Asp Leu Ser
385                 390                 395                 400

Lys Trp Leu Gly Lys Ser Val Val Leu Glu Cys Val Ser Arg Pro Asn
                405                 410                 415

Asn His Phe Ala Phe Pro Ala Tyr Ile Val Leu Ser Val Lys Glu Gly
            420                 425                 430

Ala

<210>  2
<211>  569
<212>  PRT
<213>  Chlamydia trachomatis

<400> 2

Met Pro Lys Gln Ala Asp Tyr Thr Trp Gly Ala Lys Lys Asn Leu Asp
1               5                   10                  15

Thr Ile Ala Cys Leu Pro Glu Asp Val Lys Gln Phe Lys Asp Leu Leu
            20                  25                  30

Tyr Ala Met Tyr Gly Phe Thr Ala Thr Glu Glu Glu Pro Thr Ser Glu
        35                  40                  45

Val His Pro Gly Ala Ile Leu Lys Gly Thr Val Val Asp Ile Ser Lys
    50                  55                  60

Asp Phe Val Val Val Asp Val Gly Leu Lys Ser Glu Gly Val Ile Pro
65                  70                  75                  80

Met Ser Glu Phe Ile Asp Ser Ser Glu Gly Leu Thr Val Gly Ala Glu
                85                  90                  95

Val Glu Val Tyr Leu Asp Gln Thr Glu Asp Asp Glu Gly Lys Val Val
            100                 105                 110

Leu Ser Arg Glu Lys Ala Thr Arg Gln Arg Gln Trp Glu Tyr Ile Leu
        115                 120                 125

Ala His Cys Glu Glu Gly Ser Ile Val Lys Gly Gln Ile Thr Arg Lys
    130                 135                 140

Val Lys Gly Gly Leu Ile Val Asp Ile Gly Met Glu Ala Phe Leu Pro
145                 150                 155                 160

Gly Ser Gln Ile Asp Asn Lys Lys Ile Lys Asn Leu Asp Asp Tyr Val
                165                 170                 175

Gly Lys Val Cys Glu Phe Lys Ile Leu Lys Ile Asn Val Asp Arg Arg
            180                 185                 190

Asn Val Val Val Ser Arg Arg Glu Leu Leu Glu Ala Glu Arg Ile Ser
        195                 200                 205

Lys Lys Ala Glu Leu Ile Glu Gln Ile Thr Ile Gly Glu Arg Arg Lys
    210                 215                 220

Gly Ile Val Lys Asn Ile Thr Asp Phe Gly Val Phe Leu Asp Leu Asp
225                 230                 235                 240

Gly Ile Asp Gly Leu Leu His Ile Thr Asp Met Thr Trp Lys Arg Ile
                245                 250                 255

Arg His Pro Ser Glu Met Val Glu Leu Asn Gln Glu Leu Glu Val Ile

            260                 265                 270

Ile Leu Ser Val Asp Lys Glu Lys Gly Arg Val Ala Leu Gly Leu Lys
        275                 280                 285

Gln Lys Glu His Asn Pro Trp Glu Asp Ile Glu Lys Lys Tyr Pro Pro
    290                 295                 300

Gly Lys Arg Val Arg Gly Lys Ile Val Lys Leu Leu Pro Tyr Gly Ala
305                 310                 315                 320

Phe Ile Glu Ile Glu Glu Gly Ile Glu Gly Leu Ile His Val Ser Glu
                325                 330                 335

Met Ser Trp Val Lys Asn Ile Val Asp Pro Asn Glu Val Val Asn Lys
            340                 345                 350

Gly Asp Glu Val Glu Val Val Val Leu Ser Ile Gln Lys Asp Glu Gly
        355                 360                 365

Lys Ile Ser Leu Gly Leu Lys Gln Thr Lys His Asn Pro Trp Asp Asn
    370                 375                 380

Ile Glu Glu Lys Tyr Pro Ile Gly Leu Arg Val Thr Ala Glu Ile Lys
385                 390                 395                 400

Asn Leu Thr Asn Tyr Gly Ala Phe Val Glu Leu Glu Pro Gly Ile Glu
                405                 410                 415

Gly Leu Ile His Ile Ser Asp Met Ser Trp Ile Lys Lys Val Ser His
            420                 425                 430

Pro Ser Glu Leu Phe Lys Lys Gly Asn Thr Val Glu Ala Val Ile Leu
        435                 440                 445

Ser Val Asp Lys Glu Ser Lys Lys Ile Thr Leu Gly Val Lys Gln Leu
    450                 455                 460

Thr Pro Asn Pro Trp Asp Glu Ile Glu Val Met Phe Pro Val Gly Ser
465                 470                 475                 480

Asp Ile Ser Gly Val Val Thr Lys Ile Thr Ala Phe Gly Ala Phe Val
                485                 490                 495

Glu Leu Gln Asn Gly Ile Glu Gly Leu Ile His Val Ser Glu Leu Ser
            500                 505                 510

Glu Lys Pro Phe Ala Lys Ile Glu Asp Val Leu Ser Ile Gly Asp Lys
        515                 520                 525

Val Ser Ala Lys Val Ile Lys Leu Asp Pro Asp His Lys Lys Val Ser

530                 535                 540

Leu Ser Ile Lys Glu Phe Leu Val His Gly Gly Asp Ala Gly His Asp
545                 550                 555                 560

Ala Glu Glu Glu Ser Ser Asp Arg Asp
                565

<210>  3
<211>  232
<212>  PRT
<213>  Chlamydia trachomatis

<400>  3

Met Thr Lys His Gly Lys Arg Ile Arg Gly Ile Gln Glu Thr Tyr Asp
1               5                   10                  15

Leu Ala Lys Ser Tyr Ser Leu Gly Glu Ala Ile Asp Ile Leu Lys Gln
            20                  25                  30

Cys Pro Thr Val Arg Phe Asp Gln Thr Val Asp Val Ser Val Lys Leu
        35                  40                  45

Gly Ile Asp Pro Arg Lys Ser Asp Gln Gln Ile Arg Gly Ser Val Ser
    50                  55                  60

Leu Pro His Gly Thr Gly Lys Val Leu Arg Ile Leu Val Phe Ala Ala
65                  70                  75                  80

Gly Asp Lys Ala Ala Glu Ala Ile Glu Ala Gly Ala Asp Phe Val Gly
                85                  90                  95

Ser Asp Asp Leu Val Glu Lys Ile Lys Gly Gly Trp Val Asp Phe Asp
            100                 105                 110

Val Ala Val Ala Thr Pro Asp Met Met Arg Glu Val Gly Lys Leu Gly
        115                 120                 125

Lys Val Leu Gly Pro Arg Asn Leu Met Pro Thr Pro Lys Ala Gly Thr
    130                 135                 140

Val Thr Thr Asp Val Val Lys Thr Val Ala Glu Leu Arg Lys Gly Lys
145                 150                 155                 160

Ile Glu Phe Lys Ala Asp Arg Ala Gly Val Cys Asn Val Gly Val Ala
                165                 170                 175

Lys Leu Ser Phe Asp Ser Ala Gln Ile Lys Glu Asn Val Glu Ala Leu
            180                 185                 190

Cys Ala Ala Leu Val Lys Ala Lys Pro Ala Thr Ala Lys Gly Gln Tyr
        195                 200                 205

Leu Val Asn Phe Thr Ile Ser Ser Thr Met Gly Pro Gly Val Thr Val
    210                 215                 220

Asp Thr Arg Glu Leu Ile Ala Leu
225                 230

<210>  4
<211>  192
<212>  PRT
<213>  Chlamydia trachomatis

<400>  4

Met Pro Glu Gly Glu Met Met His Lys Leu Gln Asp Val Ile Asp Arg
1               5                   10                  15

Lys Leu Leu Asp Ser Arg Arg Ile Phe Phe Ser Glu Pro Val Thr Glu
            20                  25                  30

Lys Ser Ala Thr Glu Ala Ile Lys Lys Leu Trp Tyr Leu Glu Leu Thr
        35                  40                  45

Asn Pro Gly Gln Pro Ile Val Phe Val Ile Asn Ser Pro Gly Gly Ser
    50                  55                  60

Val Asp Ala Gly Phe Ala Val Trp Asp Gln Ile Lys Met Ile Ser Ser
65                  70                  75                  80

Pro Leu Thr Thr Val Val Thr Gly Leu Ala Ala Ser Met Gly Ser Val
                85                  90                  95

Leu Ser Leu Cys Ala Val Pro Gly Arg Arg Phe Ala Thr Pro His Ala
            100                 105                 110

Arg Ile Met Ile His Gln Pro Ser Ile Gly Gly Thr Ile Thr Gly Gln
        115                 120                 125

Ala Thr Asp Leu Asp Ile His Ala Arg Glu Ile Leu Lys Thr Lys Ala
    130                 135                 140

Arg Ile Ile Asp Val Tyr Val Glu Ala Thr Gly Gln Ser Arg Glu Val
145                 150                 155                 160

Ile Glu Lys Ala Ile Asp Arg Asp Met Trp Met Ser Ala Asn Glu Ala
                165                 170                 175

Met Glu Phe Gly Leu Leu Asp Gly Ile Leu Phe Ser Phe Asn Asp Leu
            180                 185                 190

<210>  5
<211>  1005
<212>  PRT

<213>  Chlamydia trachomatis

<400>  5

Met Thr Asn Ser Ile Ser Gly Tyr Gln Pro Thr Val Thr Thr Ser Thr
1               5                   10                  15

Ser Ser Thr Thr Ser Ala Ser Gly Ala Ser Gly Ser Leu Gly Ala Ser
            20                  25                  30

Ser Val Ser Thr Thr Ala Asn Ala Thr Val Thr Gln Thr Ala Asn Ala
        35                  40                  45

Thr Asn Ser Ala Ala Thr Ser Ser Ile Gln Thr Thr Gly Glu Thr Val
    50                  55                  60

Val Asn Tyr Thr Asn Ser Ala Ser Ala Pro Asn Val Thr Val Ser Thr
65                  70                  75                  80

Ser Ser Ser Ser Thr Gln Ala Thr Ala Thr Ser Asn Lys Thr Ser Gln
                85                  90                  95

Ala Val Ala Gly Lys Ile Thr Ser Pro Asp Thr Ser Glu Ser Ser Glu
            100                 105                 110

Thr Ser Ser Thr Ser Ser Ser Asp His Ile Pro Ser Asp Tyr Asp Asp
        115                 120                 125

Val Gly Ser Asn Ser Gly Asp Ile Ser Asn Asn Tyr Asp Asp Val Gly
    130                 135                 140

Ser Asn Asn Gly Asp Ile Ser Ser Asn Tyr Asp Asp Ala Ala Ala Asp
145                 150                 155                 160

Tyr Glu Pro Ile Arg Thr Thr Glu Asn Ile Tyr Glu Ser Ile Gly Gly
                165                 170                 175

Ser Arg Thr Ser Gly Pro Glu Asn Thr Ser Gly Gly Ala Ala Ala Ala
            180                 185                 190

Leu Asn Ser Leu Arg Gly Ser Ser Tyr Ser Asn Tyr Asp Asp Ala Ala
        195                 200                 205

Ala Asp Tyr Glu Pro Ile Arg Thr Thr Glu Asn Ile Tyr Glu Ser Ile
    210                 215                 220

Gly Gly Ser Arg Thr Ser Gly Pro Glu Asn Thr Ser Gly Gly Ala Ala
225                 230                 235                 240

Ala Ala Leu Asn Ser Leu Arg Gly Ser Ser Tyr Ser Asn Tyr Asp Asp
                245                 250                 255

Ala Ala Ala Asp Tyr Glu Pro Ile Arg Thr Thr Glu Asn Ile Tyr Glu
            260                 265                 270

Ser Ile Gly Gly Ser Arg Thr Ser Gly Pro Glu Asn Thr Ser Asp Gly
        275                 280                 285

Ala Ala Ala Ala Ala Leu Asn Ser Leu Arg Gly Ser Ser Tyr Thr Thr
    290                 295                 300

Gly Pro Arg Asn Glu Gly Val Phe Gly Pro Gly Pro Glu Gly Leu Pro
305                 310                 315                 320

Asp Met Ser Leu Pro Ser Tyr Asp Pro Thr Asn Lys Thr Ser Leu Leu
                325                 330                 335

Thr Phe Leu Ser Asn Pro His Val Lys Ser Lys Met Leu Glu Asn Ser
            340                 345                 350

Gly His Phe Val Phe Ile Asp Thr Asp Arg Ser Ser Phe Ile Leu Val
        355                 360                 365

Pro Asn Gly Asn Trp Asp Gln Val Cys Ser Ile Lys Val Gln Asn Gly
    370                 375                 380

Lys Thr Lys Glu Asp Leu Asp Ile Lys Asp Leu Glu Asn Met Cys Ala
385                 390                 395                 400

Lys Phe Cys Thr Gly Phe Ser Lys Phe Ser Gly Asp Trp Asp Ser Leu
                405                 410                 415

Val Glu Pro Met Val Ser Ala Lys Ala Gly Val Ala Ser Gly Gly Asn
            420                 425                 430

Leu Pro Asn Thr Val Ile Ile Asn Asn Lys Phe Lys Thr Cys Val Ala
        435                 440                 445

Tyr Gly Pro Trp Asn Ser Gln Glu Ala Ser Ser Gly Tyr Thr Pro Ser
    450                 455                 460

Ala Trp Arg Arg Gly His Arg Val Asp Phe Gly Gly Ile Phe Glu Lys
465                 470                 475                 480

Ala Asn Asp Phe Asn Lys Ile Asn Trp Gly Thr Gln Ala Gly Pro Ser
                485                 490                 495

Ser Glu Asp Asp Gly Ile Ser Phe Ser Asn Glu Thr Pro Gly Ala Gly
            500                 505                 510

Pro Ala Ala Ala Pro Ser Pro Thr Pro Ser Ser Ile Pro Ile Ile Asn
        515                 520                 525

Val Asn Val Asn Val Gly Gly Thr Asn Val Asn Ile Gly Asp Thr Asn
    530                 535                 540

Val Asn Thr Thr Asn Thr Thr Pro Thr Thr Gln Ser Thr Asp Ala Ser
545                 550                 555                 560

Thr Asp Thr Ser Asp Ile Asp Asp Ile Asn Thr Asn Asn Gln Thr Asp
                565                 570                 575

Asp Ile Asn Thr Thr Asp Lys Asp Ser Asp Gly Ala Gly Gly Val Asn
            580                 585                 590

Gly Asp Ile Ser Glu Thr Glu Ser Ser Ser Gly Asp Asp Ser Gly Ser
        595                 600                 605

Val Ser Ser Ser Glu Ser Asp Lys Asn Ala Ser Val Gly Asn Asp Gly
    610                 615                 620

Pro Ala Met Lys Asp Ile Leu Ser Ala Val Arg Lys His Leu Asp Val
625                 630                 635                 640

Val Tyr Pro Gly Glu Asn Gly Gly Ser Thr Glu Gly Pro Leu Pro Ala
                645                 650                 655

Asn Gln Thr Leu Gly Asp Val Ile Ser Asp Val Glu Asn Lys Gly Ser
            660                 665                 670

Ala Gln Asp Thr Lys Leu Ser Gly Asn Thr Gly Ala Gly Asp Asp Asp
        675                 680                 685

Pro Thr Thr Thr Ala Ala Val Gly Asn Gly Ala Glu Glu Ile Thr Leu
    690                 695                 700

Ser Asp Thr Asp Ser Gly Ile Gly Asp Asp Val Ser Asp Thr Ala Ser
705                 710                 715                 720

Ser Ser Gly Asp Glu Ser Gly Gly Val Ser Ser Pro Ser Ser Glu Ser
                725                 730                 735

Asn Lys Asn Thr Ala Val Gly Asn Asp Gly Pro Ser Gly Leu Asp Ile
            740                 745                 750

Leu Ala Ala Val Arg Lys His Leu Asp Lys Val Tyr Pro Gly Asp Asn
        755                 760                 765

Gly Gly Ser Thr Glu Gly Pro Leu Gln Ala Asn Gln Thr Leu Gly Asp
    770                 775                 780

Ile Val Gln Asp Met Glu Thr Thr Gly Thr Ser Gln Glu Thr Val Val
785                 790                 795                 800

Ser Pro Trp Lys Gly Ser Thr Ser Ser Thr Glu Ser Ala Gly Gly Ser
                805                 810                 815

Gly Ser Val Gln Thr Leu Leu Pro Ser Pro Pro Pro Thr Pro Ser Thr
            820                 825                 830

Thr Thr Leu Arg Thr Gly Thr Gly Ala Thr Thr Thr Ser Leu Met Met
        835                 840                 845

Gly Gly Pro Ile Lys Ala Asp Ile Ile Thr Thr Gly Gly Gly Gly Arg
    850                 855                 860

Ile Pro Gly Gly Gly Thr Leu Glu Lys Leu Leu Pro Arg Ile Arg Ala
865                 870                 875                 880

His Leu Asp Ile Ser Phe Asp Ala Gln Gly Asp Leu Val Ser Thr Glu
                885                 890                 895

Glu Pro Gln Leu Gly Ser Ile Val Asn Lys Phe Arg Gln Glu Thr Gly
            900                 905                 910

Ser Arg Gly Ile Leu Ala Phe Val Glu Ser Ala Pro Gly Lys Pro Gly
        915                 920                 925

Ser Ala Gln Val Leu Thr Gly Thr Gly Gly Asp Lys Gly Asn Leu Phe
    930                 935                 940

Gln Ala Ala Ala Ala Val Thr Gln Ala Leu Gly Asn Val Ala Gly Lys
945                 950                 955                 960

Val Asn Leu Ala Ile Gln Gly Gln Lys Leu Ser Ser Leu Val Asn Asp
                965                 970                 975

Asp Gly Lys Gly Ser Val Gly Arg Asp Leu Phe Gln Ala Ala Ala Gln
            980                 985                 990

Thr Thr Gln Val Leu Ser Ala Leu  Ile Asp Thr Val Gly
        995                 1000                1005

<210>  6
<211>  195
<212>  PRT
<213>  Chlamydia trachomatis

<400>  6

Met Gly Ser Leu Val Gly Arg Gln Ala Pro Asp Phe Ser Gly Lys Ala
1               5                   10                  15

Val Val Cys Gly Glu Glu Lys Glu Ile Ser Leu Ala Asp Phe Arg Gly
            20                  25                  30

Lys Tyr Val Val Leu Phe Phe Tyr Pro Lys Asp Phe Thr Tyr Val Cys

35                  40                  45

Pro Thr Glu Leu His Ala Phe Gln Asp Arg Leu Val Asp Phe Glu Glu
    50                  55                  60

Arg Gly Ala Val Val Leu Gly Cys Ser Val Asp Asp Ile Glu Thr His
65                  70                  75                  80

Ser Arg Trp Leu Ala Val Ala Arg Asn Ala Gly Gly Ile Glu Gly Thr
                85                  90                  95

Glu Tyr Pro Leu Leu Ala Asp Pro Ser Phe Lys Ile Ser Glu Ala Phe
            100                 105                 110

Gly Val Leu Asn Pro Glu Gly Ser Leu Ala Leu Arg Ala Thr Phe Leu
        115                 120                 125

Ile Asp Lys Tyr Gly Val Val Arg His Ala Val Ile Asn Asp Leu Pro
    130                 135                 140

Leu Gly Arg Ser Ile Asp Glu Glu Leu Arg Ile Leu Asp Ser Leu Ile
145                 150                 155                 160

Phe Phe Glu Asn His Gly Met Val Cys Pro Ala Asn Trp Arg Ser Gly
                165                 170                 175

Glu Arg Gly Met Val Pro Ser Glu Glu Gly Leu Lys Glu Tyr Phe Gln
            180                 185                 190

Thr Met Asp
        195

<210>  7
<211>  829
<212>  PRT
<213>  Chlamydia trachomatis

<400>  7

Met Gly Ile Arg Leu Val Ile Asp Lys Gly Pro Leu Ser Gly Thr Val
1               5                   10                  15

Leu Ile Leu Glu Asn Gly Thr Ser Trp Ser Leu Gly Ser Asp Gly Lys
            20                  25                  30

Ala Ser Asp Ile Leu Leu Gln Asp Glu Lys Leu Ala Pro Ser Gln Ile
        35                  40                  45

Arg Ile Thr Leu Lys Asp Gly Glu Tyr Tyr Leu Glu Asn Leu Asp Ala
    50                  55                  60

Leu Arg Pro Val Ser Val Asp Gly Thr Val Ile Thr Ala Pro Val Leu
65                  70                  75                  80

Leu Lys Asp Gly Val Ser Phe Val Met Gly Ser Cys Gln Val Ser Phe
                85                  90                  95

Phe Lys Gly Glu Glu Val Glu Gly Asp Ile Glu Leu Ser Phe Gln Thr
            100                 105                 110

Glu Gly Gly Asn Glu Gly Glu Pro Ala Ala Gln Gly Ser Ser Ser Val
        115                 120                 125

Ser Ser Glu Ala Pro Lys Lys Glu Thr Gly Asn Pro Ser Leu Pro Ser
    130                 135                 140

Glu Ala Lys Ala Ser Gly Glu Val Ser Ser Ser Ala Ile Ala Lys Glu
145                 150                 155                 160

Gln Glu Leu Ala Ala Ser Phe Leu Ala Ser Val Glu Lys Glu Pro Gly
                165                 170                 175

Thr Pro Lys Glu Val Ser Glu Pro Lys Val Ser Ser Gln Glu Gly Gln
            180                 185                 190

Thr Pro Ser Val Thr Gly Glu Lys Lys Asp Leu Glu Leu Pro Leu Ala
        195                 200                 205

Ser Gln Glu Gln Pro Lys Gln Thr Thr Pro Ser Gly Ser Gly Glu Pro
    210                 215                 220

Thr Gln Ser Gln Asn Ala Ser Met Glu Glu Asn Arg Thr Ser Pro Asp
225                 230                 235                 240

Gln Asn Gln Gln Pro Gln Leu Ser Ser Ala Ser Glu Ser Gly Ser Gln
                245                 250                 255

Ser Pro Glu Asn Gln Glu Gln Gln Pro Ser Gln Thr Pro Pro Pro Ser
            260                 265                 270

Pro Glu Thr Pro Glu Pro Ser Gly Glu Pro Asn Ser Ala Thr Glu Glu
        275                 280                 285

Asn Ser Pro Ser Pro Met Glu Lys Ala Ser Val Thr Glu Glu Gly Ser
    290                 295                 300

Ser Gly Thr Ser Glu Glu Glu Lys Glu Gly Glu Glu Asp Thr Ala Glu
305                 310                 315                 320

Ser Ala Ala Asn Glu Glu Pro Lys Ala Glu Ala Ser Gln Glu Glu Glu
                325                 330                 335

Lys Lys Glu Glu Asp Lys Gly Glu Val Leu Ala Pro Phe Asn Val Gln
            340                 345                 350

Asp Leu Phe Arg Phe Asp Gln Gly Ile Phe Pro Ala Glu Ile Glu Asp
        355                 360                 365

Leu Ala Gln Lys Gln Val Ala Val Asp Leu Thr Gln Pro Ser Arg Phe
    370                 375                 380

Leu Leu Lys Val Leu Ala Gly Ala Asn Ile Gly Ala Glu Phe His Leu
385                 390                 395                 400

Asp Ser Gly Lys Thr Tyr Ile Val Gly Ser Asp Pro Gln Val Ala Asp
                405                 410                 415

Ile Val Leu Ser Asp Met Ser Ile Ser Arg Gln His Ala Lys Ile Ile
            420                 425                 430

Ile Gly Asn Asp Asn Ser Val Leu Ile Glu Asp Leu Gly Ser Lys Asn
        435                 440                 445

Gly Val Ile Val Glu Gly Arg Lys Ile Glu His Gln Ser Thr Leu Ser
    450                 455                 460

Ala Asn Gln Val Val Ala Leu Gly Thr Thr Leu Phe Leu Leu Val Asp
465                 470                 475                 480

Tyr Ala Ala Pro Ser Asp Thr Val Met Ala Thr Ile Ser Ser Glu Asp
                485                 490                 495

Tyr Gly Leu Phe Gly Arg Pro Gln Ser Pro Glu Glu Ile Ala Ala Arg
            500                 505                 510

Ala Ala Glu Glu Glu Glu Glu Lys Arg Lys Arg Ala Thr Leu Pro Thr
        515                 520                 525

Gly Ala Phe Ile Leu Thr Leu Phe Ile Gly Gly Leu Ala Leu Leu Phe
    530                 535                 540

Gly Ile Gly Thr Ala Ser Leu Phe His Thr Lys Glu Val Val Ser Ile
545                 550                 555                 560

Asp Gln Ile Asp Leu Ile His Asp Ile Glu His Val Ile Gln Gln Phe
                565                 570                 575

Pro Thr Val Arg Phe Thr Phe Asn Lys Asn Asn Gly Gln Leu Phe Leu
            580                 585                 590

Ile Gly His Val Arg Asn Ser Ile Asp Lys Ser Glu Leu Leu Tyr Lys
        595                 600                 605

Val Asp Ala Leu Ser Phe Val Lys Ser Val Asp Asp Asn Val Ile Asp
    610                 615                 620

Asp Glu Ala Val Trp Gln Glu Met Asn Ile Leu Leu Ser Lys Asn Pro
625                 630                 635                 640

Glu Phe Lys Gly Ile Ser Met Gln Ser Pro Glu Pro Gly Ile Phe Val
                645                 650                 655

Ile Ser Gly Tyr Leu Lys Thr Glu Glu Gln Ala Ala Cys Leu Ala Asp
            660                 665                 670

Tyr Leu Asn Leu His Phe Asn Tyr Leu Ser Leu Leu Asp Asn Lys Val
        675                 680                 685

Ile Ile Glu Ser Gln Val Met Lys Ala Leu Ala Gly His Leu Val Gln
    690                 695                 700

Ser Gly Phe Ala Asn Val His Val Ser Phe Thr Asn Gly Glu Ala Val
705                 710                 715                 720

Leu Thr Gly Tyr Ile Asn Asn Lys Asp Ala Asp Lys Phe Arg Thr Val
                725                 730                 735

Val Gln Glu Leu Gln Asp Ile Ala Gly Ile Arg Ala Val Lys Asn Phe
            740                 745                 750

Val Val Leu Leu Pro Ala Glu Glu Gly Val Ile Asp Leu Asn Met Arg
        755                 760                 765

Tyr Pro Gly Arg Tyr Arg Val Thr Gly Phe Ser Lys Cys Gly Asp Ile
    770                 775                 780

Ser Ile Asn Val Val Val Asn Gly Arg Ile Leu Thr Arg Gly Asp Ile
785                 790                 795                 800

Leu Asp Gly Met Thr Val Thr Ser Ile Gln Ser His Cys Ile Phe Leu
                805                 810                 815

Glu Arg Glu Gly Leu Lys Tyr Lys Ile Glu Tyr Asn Lys
            820                 825